# EUROPEAN PATENT SPECIFICATION

(11) **EP 1 970 071 B1**
(45) Date of publication and mention of the grant of the patent: **17.12.2014**
(21) Application number: 06828470.2
(22) Date of filing: 28.12.2006
(51) Int. Cl.: A61K 38/18, A61K 9/127, A61P 17/02

(54) **USE OF A TOPICAL COMPOSITION CONTAINING EPIDERMAL GROWTH FACTOR (EGF) FOR DIABETIC FOOT AMPUTATION PREVENTION**
VERWENDUNG EINER EPIDERMALEN WACHSTUMSFAKTOR (EGF) ENTHALTENDEN TOPISCHEN ZUSAMMENSETZUNG ZUR VERMEIDUNG EINER FUSSAMPUTATION BEI DIABETES
UTILISATION TOPIQUE DU FACTEUR DE CROISSANCE EPIDERMIQUE DANS DES LIPOSOMES AFIN DE PREVENIR L'AMPUTATION DU PIED DIABETIQUE

(30) Priority: 29.12.2005 CU 28205
(43) Date of publication of application: 17.09.2008
(73) Proprietor: CENTRO DE INGENIERIA GENETICA Y BIOTECNOLOGIA, Ciudad de la Habana 10600 (CU)
(72) Inventor: BETANCOURT RODRIGUEZ, Blas, Yamir, La Habana 32 300 (CU); PUPO ESCALONA, Elder, Ciudad De La Habana 11 600 (CU); PÁEZ MEIRELES, Rolando, Ciudad De La Habana 11 600 (CU); BERLANGA ACOSTA, Jorge, Amador, Ciudad De La Habana 11 600 (CU)
(74) Representative: Hatzmann, Martin
(86) International application number: PCT/CU2006/000018
(87) International publication number: WO 2007/073704

(56) References cited:
- WO-A-90/11781
- WO-A-91/01719
- WO-A-03/075949
- US-A1- 4 944 948
- US-A1- 2005 107 294
- ZHU XIER ET AL: "Studies on enhancing the repair of gangrenous skin diabetic foot by epidermal growth factor" ADVANCES OF DIABETES MELLITUS IN EAST ASIA, no. 1141, 1997, pages 241-243, XP002238063

## Description

### Field of the invention

The present invention is related to topical formulations that contain epidermal growth factor (EGF) encapsulated in or associated to deformable or conventional liposomes to be applied on the surface of and around chronic ischemic skin lesions, for preventing diabetic foot amputation.

### Background of the invention

Diabetes Mellitus and its complications is the main non-traumatic cause of lower limb amputations. This is a medical problem of increasing importance, since the incidence and prevalence of diabetes should increase as a result of population aging and sedentary lifestyles. At least 15% of diabetic patients develop chronic ulcers in their feet throughout their lifetimes, 20% of these patients are estimated to require lower limb amputation (Reiber G.E., Boyko E.J., Smith D.G. (1995) Lower extremity foot ulcers and amputations in diabetes. In: Harris M.I., Cowie C.C., Reiber G., Boyko E., Stern M., Bennett P., editors. Diabetis in America, Washington, DC: US Government Printing Office, 409-28; Moss S.E., Klein R, Klein B.E. (1992) The prevalence and incidence of lower extremity amputation in a diabetic population. Arch Int Med. 152:610-6).

Several methods have been applied to the treatment of diabetic foot patients including strict metabolic control, the prophylaxis of modifiable risk factors, debriding, use of dressings, anti-microbial treatment of infections, elimination of any pressure existing on the area of the lesion, use of skin implants, growth factors and revascularization methods in case that they are indicated.

Upon strict metabolic control, the more important step to heal diabetic ulcers is debriding, and it has to be carried out before any other modality of local treatment. It consists of the removal of dead or infected tissue (including bones) that may exist within the area of the lesion and the surrounding callous tissue.

The use of dressings for diabetic foot ulcers is well established, and although several kinds of dressings have been studied, the advantages of each kind of dressing over the others are not known. Besides, since studies on the use of dressings have been few, and they have been directed mainly to low-grade ulcers, more evidences from clinical trials are required to demonstrate their efficacy. New kinds of dressings, which have been studied in clinical trials, include those based on a semi permeable polymeric membrane, promogram (a collagen matrix), alginate, carboxymethylcellulose, hyaluronan and those that use sub-atmospheric pressure (Eldor R., et al. (2004) New and experimental approaches to treatment of diabetic foot ulcers: a comprehensive review of emerging treatment strategies. Diabet Med. 21 (11):1161-73).

Adjuvant treatments have also been employed, such as the hemorrheologic and vasoactive therapies, which have provided some beneficial effect no only in the chronic stage of the ulcer but also in its re-aggravations, however, these treatments have not been generalized to the treatment of the diabetic foot. The hemorrheologic therapy is based on the demonstrated prevalence in the diabetic patient of hemorrheologic alterations and their enhancing effect on infections. On the other hand, the vasoactive therapy has been used in local perfusion alterations, which are caused by macro- or micro angiopathy, in which some prostanoids act at the tissular level.

Platelet aggregation inhibitors and thrombolytic agents have been used for some conditions. On the other hand, the revascularization technique in the ischemic patient (diabetic or non-diabetic) is risky, expensive, and is not applicable to all patients. Its indication is very limited, just as is the endovascular surgery, which has shown viability limitations, not only in the Aorto-Iliac but also in the Femoral-Popliteal arterial sectors, attributable to calcification and increased lesion sectorization. Another invention for the treatment of extensive acute skin lesions, such as venous ulcers, has been the creation of artificial human skin substitutes. However, the information on their application to ischemic diabetic foot ulcers is limited, and it is not conceivable that these products can control the ischemic process that underlies as a cause of the failure of cicatrisation (New Skin for Old. Developments in Biological Skin Substitutes. Arch Dermatol. 1998 134:344-348).

Another alternative has been the application of growth factors. Recently, the United States Food and Drug Administration (FDA), approved the use of the human recombinant platelet-derived growth factor (PDGF), to stimulate the cicatrisation of diabetic neurophatic foot ulcers.

However, the most recent randomized, double-blind, multicenter clinical trial, in which a gel formulation of PDGF was applied onto the surface of lesions, demonstrates only an efficacy of 50% (Wieman T.J., Smiell J.M., Su Y. (1998) Efficacy and safety of a topical gel formulation of recombinant human platelet-derived growth factor-BB (becaplermin) in patients with chronic neuropathic diabetic ulcers. A phase III randomized placebo-controlled double-blind study. Diabetes Care. 21 (5):822-7). In addition, the lesions treated in this clinical trial were only up to a grade of III and IV, with a normal arterial blood supply. Furthermore, the rate of recurrence observed in this study (30% in three months) was high.

The most satisfactory results on the treatment of neuropathic diabetic foot ulcers with epidermal growth factor (EGF) has been described by Berlanga, J. et al. (WO 03/053458 A1). WO-A-03075949 discloses a method for treating grade 1-2 diabetic ulcers with EGF, wherein EGF might be combined with liposomes. This invention demonstrates a high efficacy (greater than 50%) of treatment of higher-grade (IV and V) lesions with a severe ischemic component, thus preventing diabetic foot amputation. In this clinical trial, EGF was infiltrated in the lesions and around them by using hypodermic needles. This infiltration method is very effective for EGF administration because it can deliver a high amount of EGF directly in the viable tissue. However, the injection has the disadvantage that it causes pain; as a result of which some patients may abandon the treatment, and it has the risk of producing sepsis.

Liposomes have been proposed for use in a variety of topical applications since they facilitate the absorption of drugs through the skin, decrease drug-associated toxicity and provide a sustained release of drugs for a longer period of time. The colloidal properties of liposomes, which determine their drug transport efficiency by the topical route, depend to a large extend, among other factors, on their lipid composition. Generally, traditional liposomes comprised of phospholipids and sterols have been found to modulate the penetration of drugs through the skin since these vesicles, although not capable of penetrating the viable skin, may accumulate in the stratum corneum and other layers located closer to the surface of the skin (Barry B.W. (2001) Novel mechanisms and devices to enable successful transdermal drug delivery. Eur J Pharm Sci. 14(2):101-14; van den Bergh, B.A.I., de Vries, I.S., Bouwstra, J.A., (1998) Interactions between liposomes and human stratum corneum studied by freeze-substitution electron microscopy. Int. J. Pharm. 167, 57-67). When tensoactive agents, such as detergents, are included in liposomes, these vesicles display a greater deformability (deformable liposomes) which can penetrate, in an intact form and more efficiently, trough the pores of the stratum corneum into deeper viable skin layers (Cevc G., Schatzlein A., Richardsen H. (2002) Ultradeformable lipid vesicles can penetrate the skin and other semi-permeable barriers unfragmented. Evidence from double label CLSM experiments and direct size measurements. Biochim. Biophys. Acta 1564(1):21-30; Cevc G. (2004) Lipid vesicles and other colloids as drug carriers on the skin. Adv Drug Deliv Rev. 56(5):675-711). However, the usefulness of traditional liposomes, comprised of phospholipids and sterols, for administering EGF by the topical route has been demonstrated in applications that notably differ in their nature from those described in the present invention. In particular, Uster, P.S. et al. (US 4944948) reported that the association of EGF to a liposome dispersion of high viscosity could prolong the local concentration of EGF at the application site in rabbits. Also, it has been shown that a formulation of EGF with Su Yu Ping, of which liposome is the main component, enhance, as compared to EGF alone, the healing of grade II burn wounds in patients when applied by the topical route (Liao Y., Guo L., Ding E.Y. (2003) A comparative study on burn wound healing treated by different methods of recombinant human epidermal growth factor. Zhongguo Xiu Fu Chong Jian Wai Ke Za Zhi 17(4):301-2). On the other hand, it has not been described or proposed the use of deformable liposomes for applying EGF topically on chronic ischemic cutaneous lesions of high amputation risk in diabetic foot patients. Despite that it has been shown that these liposomes, loaded with active principles other than EGF, can penetrate the normal skin more efficiently, it cannot be deduced from those experiences whether these vesicles could transport EGF with enough efficacy to regions deep below neurophatic and ischemic chronic cutaneous lesions of grade IV or V so as to allow their healing and the prevention of foot amputation. Since the structure of the skin within those types of lesions is very different from that of normal skin it is conceivable that the penetrability and integrity of liposomes within such kind of lesions might be appreciable affected. Also, since the tissue of those kind of diabetic foot lesions contain a level of proteolytic enzymes much higher than that of normal skin (Yager D.R., Chen S.M., Ward S.I., et al. (1997) Ability of chronic wound fluids to degrade peptide growth factors is associated with increased levels of enastase activity and disminished levels of proteinase inhibitors. Wound Rep. Reg. 5:23-32) the active pharmaceutical compositions may be enzimatically degraded to a larger extend.

Therefore, an effective formulation that contains EGF, for the treatment of chronic ischemic diabetic foot ulcers, which achieves a high bioavailability of EGF in the tissue deep below the lesion, similar to that obtained by EGF infiltration with hypodermic needles, which prevents the amputation of the extremity and at the same time, avoids the pain and the risk of infection associated to the injection, has not been demonstrated yet.

### Detailed description of the invention.

The present invention solves the aforementioned problem by the use of topical formulations that contain EGF encapsulated or associated to deformable or traditional liposomes, which provide a high bioavailability of EGF in the tissue deep below the cutaneous lesions, similar to that obtained by infiltrating EGF with hypodermic needles. These topical formulations are applied on the surface and around chronic ischemic cutaneous lesions and have the advantage that they avoid the pain of injection and the associated risk of infection.

Another aspect of the present invention is related to a topical pharmaceutical formulation, which contains an effective amount of EGF encapsulated in or associated to deformable or conventional liposomes, wherein the liposome is comprised of one or more pharmaceutically acceptable lipids. When EGF is applied by using these formulations it is efficiently transported to the viable tissue deep below the lesions and successfully protected against protease-mediated degradation, this is very important as these enzymes are in high levels in this kind of diabetic foot lesions.

In a preferred embodiment the effective amount of EGF is from 0.025 to 0.075 mg / gram of substance. These formulations allow the irreversible cicatrisation of chronic cutaneous lesions, thus preventing amputation practice which otherwise would be the only alternative for the ischemic extremity.

In another preferred embodiment, the liposome of the topical pharmaceutical formulation is comprised by one or more pharmaceutically acceptable lipids selected from the group consisting of a neutral lipid, a negatively charged lipid, a positively charged lipid, a lipid conjugated to polyethyleneglycol or a lipid conjugated to a carbohydrate. Such liposomes may also be comprised of one or more pharmaceutically acceptable lipids and one or more non-ionic, zwitterionic, anionic or cationic surfactant agents.

### Brief description of the drawings

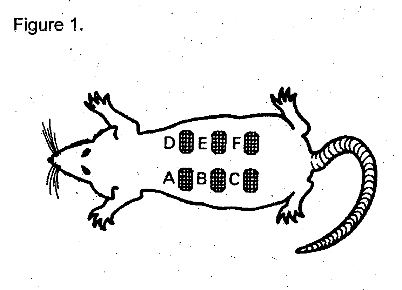

**Figure 1****.** Location of ulcers induced in the animal model

### Detailed description of the embodiments / Examples

### Example 1. Manufacturing of EGF-loaded liposomes.

Phosphatidylcholine, at a concentration of 10 mg/mL, was dissolved in absolute ethanol in a 50 mL round-bottomed flask. The solvent was removed by rotary-evaporation until a dried lipid film was formed on the walls of the flask. To encapsulate EGF into liposomes, the dried lipid film was hydrated with a buffered aqueous solution containing EGF and homogenized by agitation. To decrease the size of the vesicles, the suspension was subjected to several extrusion passes through a 100-nm-pore-size polycarbonate membrane until the average size of the vesicles was about 100 nm. The suspension of EGF-loaded liposomes was centrifuged at 100 000 × *g* for 40 min at 4°C to separate non-encapsulated from encapsulated EGF. The supernatant was transferred to a fresh tube and the pellet was resuspended in phosphate-buffered saline at pH 7.2. The centrifugation step was repeated once more under the same conditions and the supernatant was transferred to a fresh tube and mixed with the supernatant of the first centrifugation step. The pellet (containing EGF-loaded liposomes) was resuspended in phosphate-buffered saline at pH 7.2. This final preparation was stored at 4°C until use.

### Manufacturing of EGF-loaded deformable liposomes.

A total of 85.8 mg of phosphatidylcholine and 11.7 mg of sodium deoxycholate were dissolved in 100 µL warm absolute ethanol, diluted with 900 µL phosphate buffer, and vortexed until a homogenous suspension of milky appearance was obtained. This liposomal preparation was to several extrusion passes through a 100-nm-pore-size polycarbonate membrane until the average size of the vesicles was about 100 nm. To load EGF into the deformable liposomes, 100 µL of the liposomal suspension were mixed with 25 µL of a solution containing different (250, 500 or 1000 µg) amounts of EGF and the mixtures were incubated for 24 h at 4°C.

### Example 2. Determination of the encapsulation efficiency of EGF into liposomes.

To determine the incorporation efficiency of EGF into liposomes, liposome suspensions were centrifuged at high speed and the protein content of the obtained pellet (EGF-loaded liposomes) and supernatant (free EGF) was determined. The suspension of liposomes was centrifuged at 100 000 × *g* for 40 min at 4°C. The supernatant was transferred to a fresh tube and the pellet was resuspended in phosphate-buffered saline at pH 7.2. The centrifugation step was repeated once more under the same conditions and the supernatant was transferred to a fresh tube and mixed with the supernatant of the first centrifugation step. The pellet (containing EGF-loaded liposomes) was resuspended in 500 µL phosphate-buffered saline at pH 7.2. Then, the protein of the pellet or the supernatant was extracted and separated from the lipids by adding 0.5% (v/v) Triton X-100 to the samples and subjecting them to reverse-phase high-performance liquid chromatography (RP-HPLC). The protein content was determined on the basis of the area under the curve of the chromatogram obtained by RP-HPLC at an absorbance wavelength of 226 nm. The incorporation efficiency of EGF into liposomes was determined as the ratio of the protein content of the pellet (EGF-loaded liposomes) to the total protein content added at the beginning of the encapsulation process x 100%.

**Table 1. Incorporation efficiency of EGF into liposomes.**

| Amount of EGF added (µg) | Incorporation efficiency (%) (Ave ± STDEV, *n*=3) | |
|---|---|---|
| | Traditional liposomes | Deformable liposomes |
| 250 | 7.6 ± 1.2 | 18.2 ± 1.5 |
| 500 | 10.4 ± 0.8 | 25.7 ± 3.3 |
| 1000 | 8.2 ± 1 | 23.4 ± 5.2 |

### Example 3. Determination of the size and morphology of liposomes.

Liposome samples were analyzed by transmission electron microscopy to determine the size and morphology of liposomes. Liposomes were visualized by negative staining with uranyl acetate. The negatively stained samples were observed under a transmission electron microscope Jeol-JEM 2000EX operating at 80 KV. The electron micrographs corresponding to each liposome preparation were digitalized by using a scanner and the diameter of liposomes was measured by using the DIGIPAT Version 3.3 software (EICISOFT, Havana, Cuba). The particle size was averaged over the total number of liposomes present in each micrograph and was expressed as the mean ± the standard deviation of three independent determinations.

The preparations of deformable or traditional liposomes loaded with EGF were comprised of a homogenous population of vesicles of spherical or ellipsoidal shape. The average size of vesicles was 130 ± 7 nm and 123 ± 4 nm, for traditional and deformable liposomes, respectively.

### Example 4. Manufacturing of a gel formulation containing EGF-loaded liposomes.

The suspension of deformable or traditional liposomes loaded with EGF was diluted 1.5-fold in phosphate buffer pH 7.2, and Carbomer (Carbopol 940) buffered at pH 7.2 was added at a final concentration of 1.25% (w/v). This formulation also contained 0.02% (w/v) Butyl hydroxytoluene (BHT), 0.1% (w/v) EDTA, 0.25% (w/v) methyl parahydroxybenzoate, 0.525% (w/v) benzylic alcohol, 0.2% (p/v) sodium hydroxide and 3% (w/v) glycerol.

Gel formulations of traditional and deformable liposomes had a viscosity of approximately 840 mPa s and 730 mPa s, respectively.

### Example 5. Demonstration of the protecting effect of EGF encapsulation into liposomes against EGF enzymatic degradation in vitro.

This experiment was aimed at evaluating if the encapsulation of EGF into traditional or deformable liposomes is advantageous to preserve the integrity of EGF in the proteolytic environment of the diabetic foot tissue. To this aim, biopsies that had been taken from foot ulcer tissues of diabetic patients after local anesthesia were resuspended in phosphate-buffered saline pH 7.2. A total of 25 µg of free EGF or EGF encapsulated into liposomes was added to the samples and incubated for 20, 40, o 60 min at 37°C. Then, 50% (v/v) acetic acid was added to stop the reaction. After adding 5% (v/v) Triton X-100, the samples were centrifuged at 4000 rpm for 15 min and filtered through polycarbonate filters with a pore size of 0.2 or 0.45 µm. The content of EGF of the samples was quantified by correlation with the area under the curve of the chromatogram obtained at 226 nm by RP-HPLC. The percent of EGF remaining after the incubation was calculated as the ratio of the content of EGF remaining in the samples after the incubation to the content of EGF added initially x 100%. The data presented in Table 2 evidence that, after exposure to diabetic foot biopsies, the samples containing EGF encapsulated into liposomes (traditional or deformable) preserve a higher amount of intact EGF than that of the samples containing non-encapsulated EGF.

**Tabla 2. EGF remaining after its incubation with biopsies of foot ulcer tissues from diabetic patients.**

| | **Remaining EGF (%)** | | |
|---|---|---|---|
| | **20 min** | **40 min** | **60 min** |
| **EGF encapsulated in traditional liposomes** | 95.1 ± 1.1 | 83.4 ± 4.7 | 58.6 ± 3.4 |
| **EGF encapsulated in deformable liposomes** | 93.4 ± 2.1 | 80.6 ± 3.8 | 55.9 ± 2.5 |
| **Free EGF** | 82.5 ± 2.5 | 66.9 ± 3.6 | 37.8 ± 1.3 |

### Example 6. Demonstration of the efficacy of EGF-containing formulations in experimental models of acute and controlled torpid lesions.

### Experimental model of controlled acute lesions.

The following experiment was performed with the aim of evaluating the cicatrisation effect of the new pharmaceutical formulation for topical use, which is based on EGF-loaded (deformable or traditional) liposomes, in acute lesions of satisfactory prognosis.

Experimental Biombdel: Male Wistar rats with a body weight of 225-250 grams. Animal were maintained in controlled areas of the animal facility at CIGB under a constant illumination schedule of 12 x 12 hours, air change cycles, and free access to the diet. The rats were individually housed into T3 boxes with replacement of the bedding every 48 hours after its sterilization.

Induction of ulcers: Animals were anesthetized by intraperitoneal injection of ketamine/xylazine. The back of rats comprising the area from the retroscapular space up to the sacrum was mechanically and chemically depilated. This region was aseptisized with a solution of povidone-iodine and isopropyl alcohol. The area over the skin selected for the induction of ulcers was marked with Chinese ink in order to induce circular, total width lesions with the aid of 9 mm-diameter biotomes (AcuDrem, FI, USA). As indicated in Figure 1, six symmetric and equidistant lesions were induced in each animal. Lesions were washed with a sterile saline solution and their inner border was delineated with permanent ink for later calculation of wound area at time zero. The lesions of all animals were hygienized daily with 70% ethanol and sterile saline before the application of any treatment.

### Experimental groups:

The ulcers created in the animals were randomly assigned to the following experimental treatment groups by using an input order/group crossmatching table:
Group I- no treatment.
Group II- placebo (formulation of empty deformable liposomes- without EGF) applied topically.
Group III- infiltration of an EGF solution containing 75 micrograms per milliliter by using hypodermic needles. Infiltrations were performed on the border and the bottom of wounds
Group IV- treatment with the formulation of traditional liposomes containing 25 micrograms of EGF per gram of substance applied topically
Group V- treatment with the formulation of traditional liposomes containing 75 micrograms of EGF per gram of substance applied topically
Group VI- treatment with the formulation of deformable liposomes containing 25 micrograms of EGF per gram of substance applied topically
Group VII- treatment with the formulation of deformable liposomes containing 75 micrograms of EGF per gram of substance applied topically

Each group consisted of 10 rats; therefore 60 wounds per group were studied. Treatments were performed daily, after sedation of animals with diazepam administrated by the intra-peritoneal route.

### Determination of the level of wound closure. Histologic processing:

The lesions were traced on transparent acetate sheets for calculating the kinetics of the wounds contraction over the following times: Time 0- represents 100% of opened lesion area and 0% wound contraction, Time 1- 72 hours after the induction of lesions, Time 2- five days after the induction of lesions, Time 3- seven days after the induction of lesions, Time 4- nine days after the induction of lesions. The ninth day was set as the end of this study, on which the animals were sacrificed, according to previous experiences on the kinetics of the spontaneous cicatrisation of these lesions. The images with traced lesions borders were digitized. The area of lesions and the percent of contraction were calculated by using the image analysis software DIGIPAT. Statistical analyses of each parameter were performed with the SPSS package by using the non-parametric Mann Whitney U test, a signification level of p<0.05 was assumed.

Animals were sacrificed by intra-peritoneal injection with an overdose of sodium pentobarbital (250 mg/kg). Lesion were dried up from the panniculus carnosus and fixed in 10% neutral formalin for later inclusion in paraffin. The hematoxylin/eosin, van Giesson's and Masson's trichromic stains were used. For each group, the number of animals with 100% epithelization of the lesion, and with a stratified and differentiated epidermis was determined. The kinetics of wound contraction is shown in Table 3 (contraction values in mm are expressed in terms of percent change of wound size with respect to wound size at Time 0).

**Table 3. Contraction kinetic values of controlled acute ulcers.**

| | **Contraction (%)** | | | | |
|---|---|---|---|---|---|
| Groups | Time 0 | Time 1 | Time 2 | Time 3 | Time 4 |
| Group I | 0 | 5.3 ± 1.8 | 9.6 ± 1.1 | 31.5 ± 2 | 59.8 ± 2.1 |
| Group II | 0 | 6.1 ± 1.2 | 9.3 ± 0.7 | 30.2 ± 1.6 | 61.2 ± 3 |
| Group III | 0 | 12.3 ± 2.1*^{a} | 23 ± 2.7*^{a} | 67.5 ± 3.1*^{a} | 76.5 ± 2.2*^{a} |
| Group IV | 0 | 14.6 ± 1.8*^{b} | 31.6 ± 1.2** | 74.5 ± 2.4** | 86.2 ± 1.7** |
| Group V | 0 | 15.9 ± 1.5*^{b} | 33.2 ± 1.9** | 78.2 ± 4.1** | 88.6 ± 2.2** |
| Group VI | 0 | 16.1 ± 1.7*^{b} | 35.4 ± 2.5** | 81.7 ± 3.6** | 90.5 ± 1.3** |
| Group VII | 0 | 16.5 ± 1.6*^{b} | 36.7 ± 1.9** | 85.2 ± 4.1** | 94.3 ± 1.8** |

| | | | | | |
|---|---|---|---|---|---|
| (*^{a}) This means a statistical difference of p<0.05 with respect to groups I and II. (*^{b}) This means a statistical difference of p<0.05 with respect to groups I, II and III. (**)This means a statistical difference of p<0.01 with respect to groups I, II and III. Mann-Whitney U test. | | | | | |

As shown in Table 3, liposome-based formulations provided the most potent contraction effect on wound borders, in other words, it means that liposomal formulations produced the most favorable enhancing effect on total cicatrisation, since the contraction represent the convergence of several consolidated events that approximate the wound to the remodeling phase.

The percent of area covered by mature and organized granulation tissue for each experimental group is presented in Table 4. The calculation of this parameter was carried out using the samples collected at Time 6, by counting in each sample the number of microscope fields simultaneously positive to van Giesson's and Masson's trichromic staining reactions. Two pathologists carried out these evaluations in an independent and blind manner.

**Tabla 4. Percent of granulated area at Time 4 for each experimental group.**

| | (% area covered by mature granulation tissue) |
|---|---|
| Group I | 53.2 ± 3.15 |
| Group II | 51.8 ± 2.6 |
| Group III | 66.7 ± 3.22 |
| Group IV | 89.8 ± 3.14 |
| Group V | 91.2 ± 1.63 |
| Group VI | 94.8 ± 2.08 |
| Group VII | 96.1 ± 1.75 |

| | |
|---|---|
| Study of 60 wounds per group using collagen-fibers positive reactions. | |

As shown in Table 4, the treatment of lesions with liposome-based formulations exert the most potent effect on the process of maturation and establishment of the granulation tissue, this result is in agreement with that already described on the contraction of wounds.

The effect of the treatments on the process of epithelization of lesions was also studied. The aspect of the stratified epithelium was evaluated, considering the re-epithelization of the ulcer, the presence of a stratified epithelium, and the existence of a keratin stratum. To study the lesions under the microscope, they were subjected to a longitudinal central hemisection and included in the same paraffin block. A total of 120 histological sections were studied, which corresponded to 60 lesions. The results of this study are presented in Table 5.

**Table 5. Effect of the treatments on wound epithelization.**

| | No. wounds with 100% epithelization | No. wounds with a mature epithelium |
|---|---|---|
| Group I | 27 | 22 |
| Group II | 29 | 26 |
| Group III | 37 | 25 |
| Group IV | 53 | 45 |
| Group V | 55 | 47 |
| Group VI | 55 | 48 |
| Group VII | 57 | 50 |

As shown in Table 5, groups IV, V, VI and VII treated with liposomal formulations displayed the best indicators of the epithelial response, given by the total re-epithelization and maturity of the epithelium.

It can be concluded therefore that the treatment of lesions with the liposomal formulations favors (*i*) the process of contraction of acute controlled wounds; (*ii*) the granulation process and its maturation; the epidermal re-epithelization and differentiation process and (*iv*) these processes were not associated to the formation of aberrant granulation tissue, granulomas or foreign bodies.

### Experimental model of chronic cutaneous ulcers.

The following experiment was aimed at evaluating the cicatrisation effect of the new pharmaceutical formulation of topical use that is based on traditional or deformable liposomes containing EGF in chronic lesions of poor prognosis, which simulate lesions in diabetic patients.

Experimental Biomodel: Male Wistar rats with a body weight of 225-250 grams. Animal were maintained in controlled areas of the animal facility at CIGB under a constant illumination schedule of 12 x 12 hours, air change cycles, and free access to the diet. The rats were individually housed into T3 boxes with replacement of the bedding every 48 hours after its sterilization. The animals had previously been treated for two months with a 0.01% methylglyoxal solution to create a glycosylation environment similar to that occurring in a diabetic patient of long term evolution. Among other organic damages, this leads to a slow down of the granulation and remodelation of wounds (Berlanga J., Cibrian D., et al. (2005) Methylglyoxal administration induces diabetes-like microvascular changes and perturbs the healing process of cutaneous wounds. Clin Sci (Lond). 109(1):83-95).

Induction of ulcers: Animals were anesthetized by intraperitoneal injection of ketamine/xylazine. The back of rats comprising the area from the retroscapular space up to the sacrum was mechanically and chemically depilated. This region was aseptisized with a solution of povidone-iodine and isopropyl alcohol. The area over the skin selected for the induction of ulcers was marked with Chinese ink in order to induce circular, total width lesions with the aid of 9 mm-diameter biotomes (AcuDrem, FI, USA). As indicated in Figure 1, six symmetric and equidistant lesions were induced in each animal. Lesions were washed with a sterile saline solution and their inner border was delineated with permanent ink for later calculation of wound area at time zero. The lesions of all animals were hygienized daily with 70% ethanol and sterile saline before the application of any treatment.

### Experimental groups:

The ulcers created in the animals were randomly assigned to the following experimental treatment groups by using an input order/group crossmatching table:
Group I- no treatment.
Group II- placebo (formulation of empty deformable liposomes- without EGF) applied topically.
Group III- infiltration of an EGF solution containing 75 micrograms per milliliter by using hypodermic needles. Infiltrations were performed on the border and the bottom of wounds
Group IV- treatment with the formulation of traditional liposomes containing 25 micrograms of EGF per gram of substance applied topically
Group V- treatment with the formulation of traditional liposomes containing 75 micrograms of EGF per gram of substance applied topically
Group VI- treatment with the formulation of deformable liposomes containing 25 micrograms of EGF per gram of substance applied topically
Group VII- treatment with the formulation of deformable liposomes containing 75 micrograms of EGF per gram of substance applied topically

Each group consisted of 10 rats; therefore 60 wounds per group were studied. Treatments were performed daily, after sedation of animals with diazepam administrated by the intra-peritoneal route.

### Determination of the level of wound closure. Histologic processing:

The lesions were traced on transparent acetate sheets for calculating the kinetics of the wounds contraction over the following times: Time 0- represents 100% of opened lesion area and 0% wound contraction, Time 1- 72 hours after the induction of lesions, Time 2- five days after the induction of lesions, Time 3- seven days after the induction of lesions, Time 4- nine days after the induction of lesions. The ninth day was set as the end of this study, on which the animals were sacrificed, according to previous experiences on the kinetics of the spontaneous cicatrisation of these lesions. The images with traced lesions borders were digitized. The area of lesions and the percent of contraction were calculated by using the image analysis software DIGIPAT. Statistical analyses of each parameter were performed with the SPSS package by using the non-parametric Mann Whitney U test, a signification level of p<0.05 was assumed.

Animals were sacrificed by intra-peritoneal injection with an overdose of sodium pentobarbital (250 mg/kg). Lesion were dried up from the panniculus carnosus and fixed in 10% neutral formalin for later inclusion in paraffin. The hematoxylinleosin, van Giesson's and Masson's trichromic stains were used. For each group, the number of animals with 100% epithelization of the lesion, and with a stratified and differentiated epidermis was determined. The kinetics of wound contraction is shown in Table 3 (contraction values in mm are expressed in terms of percent change of wound size with respect to wound size at Time 0).

**Table 6. Contraction kinetic values of chronic ulcers.**

| Contraction (%) | | | | | |
|---|---|---|---|---|---|
| Groups | Time 1 | Time 2 | Time 3 | Time 4 | Time 6 |
| Group I | 3.1 ± 1.1 | 4.1 ± 1.15 | 5.23 ± 1.3 | 11.5 ± 2.6 | 16.8 ± 2.4 |
| Group II | 4.1 ± 2.2 | 3.77 ± 1.6 | 6.3 ± 1.13 | 12.2 ± 1.04 | 18.6 ± 1.6 |
| Group III | 6.55 ± 1.14 | 10.25 ± 3.1* | 25 ± 2.7* | 53.5 ± 3.5* | 61.7 ± 2.14* |
| Group IV | 13.7 ± 0.9** | 24.2 ± 0.78** | 30.3 ± 2.4** | 68.6 ± 1.08** | 80.3 ± 1.87** |
| Group V | 14.6 ± 1.6** | 25.8 ± 0.81** | 31.4 ± 3.2** | 69.5 ± .3.4** | 83.2 ± 2.12** |
| Group VI | 15.3 ± 2.3** | 27.3 ± 1.1** | 33 ± 2.5** | 70.52 ± 2.2** | 83 ± 3.14** |
| Group VII | 16.7 ± 1.8** | 28.6 ± 0.95** | 35 ± 3.8** | 73.7 ± 2.15** | 86 ± 4.1** |

| | | | | | |
|---|---|---|---|---|---|
| (*)This means a statistical difference of p<0.05 with respect to groups I and II. (**) This means a statistical difference of p<0.01 with respect to groups I, II and III. Mann-Whitney U test. | | | | | |

As shown in Table 6, liposome-based formulations provided the most potent contraction effect on wound borders, in other words, it means that liposomal formulations produced the most favorable enhancing effect on total cicatrisation, since the contraction represent the convergence of several consolidated events that approximate the wound to the remodeling phase. Note that these experimental wounds simulate the biochemical microenvironment of diabetic wounds in which the contraction mechanism is partially or completely abolished pathologically.

### Determination of the concentration of EGF in the viable tissue located deep below the cutaneous lesions

To determine the concentration of EGF, histological cuts of the tissue deep below (1 cm) the cutaneous lesions were performed 20 min, 1 h, 4 h, 8 h, 16 h and 24 h after the induction of lesions. The tissue was homogenized into a phosphate-buffered saline solution. The concentration of EGF was determined by an Enzyme Linked Immunosorbent Assay (ELISA). As shown in Table 6a, liposome-based EGF formulations provided the transport of high amounts of EGF (experimental groups IV, V, VI and VII) to tissue regions deep below the lesions, similar to those obtained by infiltrating EGF with the aid of hypodermic needles (experimental group III).

**Tabla 6a. Concentration of EGF in the viable tissue deep below cutaneous lesions.**

| | **EGF concentration (µg/ml)** | | | | | |
|---|---|---|---|---|---|---|
| **Groups** | **20 min** | **1 h** | **4 h** | **8 h** | **16 h** | **24 h** |
| Group I | 0.01 ± 0.003 | 0.012 ± 0.005 | 0.008 ± 0.002 | 0.009 ± 0.003 | 0.007 ± 0.002 | 0.008 ± 0.003 |
| Group II | 0.013 ± 0.002 | 0.01 ± 0.001 | 0.012 ± 0.005 | 0.009 ± 0.002 | 0.01 ± 0.003 | 0.008 ± 0.001 |
| Group III | 27.55 ± 0.14* | 5.47 ± 0.1* | 1.55 ± 0.1* | 0.26 ± 0.04* | 0.07 ± 0.01* | 0.02 ± 0.005* |
| Group IV | 1.1 ± 0.4* | 2.8 ± 0.2* | 3.9 ± 0.2** | 9.1± 0.3** | 2.9 ± 0.1** | 0.08 ± 0.01** |
| Group V | 5.6 ± 0.7* | 8.2 ± 0.3* | 11.3 ± 0.5** | 20.1 ± 1.6** | 9.6 ± 1.7** | 0.23 ± 0.05** |
| Group VI | 1.5 ± 0.3* | 4.3 ± 0.7* | 7.7 ± 0.2** | 11.1 ± 0.8** | 4.6 ± 0.2** | 0.1 ± 0.03** |
| Group VII | 8.8 ± 0.4* | 12.7 ± 1.1* | 19.5 ± 2.9** | 28.6 ± 1.6** | 13.2 ± 2.1** | 0.42 ± 0.06** |

| | | | | | | |
|---|---|---|---|---|---|---|
| (*)This means a statistical difference of p<0.05 with respect to groups I and II. (**) This means a statistical difference of p<0.01 with respect to groups I, II and III. Mann-Whitney U test. | | | | | | |

In Table 7, the percent of area covered by mature and organized granulation tissue for each experimental group is presented. The calculation of this parameter was carried out using the samples collected at Time 4, by counting in each sample the number of microscope fields simultaneously positive to van Giesson's and Masson's trichromic staining reactions. Two pathologists carried out these evaluations in an independent and blind manner.

As shown in Table 7, the treatment of lesions with liposome-based formulations exert the most potent effect on the process of maturation and establishment of the granulation tissue, this result is in agreement with that already described on the contraction of wounds.

**Table 7. Percent of granulated area at Time 4 for each experimental group.**

| | (% area covered by mature granulation tissue) |
|---|---|
| Group I | 33.1 ± 4 |
| Group II | 28.6 ± 1.16 |
| Group III | 41.7 ± 2.25 |
| Group IV | 63.8 ± 2.3 |
| Group V | 65.1 ± 1.8 |
| Group VI | 68.7 ± 2.2 |
| Group VII | 69.6 ± 1.15 |

| | |
|---|---|
| Study of 60 wounds per group using collagen-fibers positive reactions. | |

The effect of the treatments on the process of epithelization of lesions was also studied. The aspect of the stratified epithelium was evaluated, considering the re-epithelization of the ulcer, the presence of a stratified epithelium, and the existence of a keratin stratum. To study the lesions under the microscope, they were subjected to a longitudinal central hemisection and included in the same paraffin block. A total of 120 histological sections were studied, which corresponded to 60 lesions. It was not necessary to eliminate any lesion due to bacterial contamination. The results of this study are presented in Table 8.

**Table 8. Effect of the treatments on wound epithelization.**

| | No. wounds with 100% epithelization | No. wounds with a mature epithelium |
|---|---|---|
| Group I | 0 | 0 |
| Group II | 0 | 0 |
| Group III | 16 | 11 |
| Group IV | 29 | 24 |
| Group V | 31 | 27 |
| Group VI | 33 | 28 |
| Group VII | 35 | 30 |

As shown in Table 8, groups IV, V, VI and VII treated with liposome-based formulations displayed the best indicators of the epithelial response, given by the total re-epithelization and maturity of the epithelium.

It can be concluded that the treatment with the liposomal formulations favors (*i*) the process of contraction of chronic wounds which accurately simulate the biochemical microenvironment of diabetic patient ulcers; (*ii*) the granulation process and its maturation. Remarkably, the treatment allows the establishment of a neoformed vascular network and (*iii*) stimulates the epidermal re-epithelization and differentiation process of lesions that are usually resistant to re-epithelization.

### Example 7. Demonstration of the efficacy of EGF-loaded liposomal topical formulations in patients with advanced diabetic foot ulcers.

The administration on grounds of compassion of the traditional or deformable liposome-based topical formulations containing 25 or 75 micrograms of EGF per gram of substance in patients with advanced diabetic foot ulcers has shown results that are similar to those obtained by EGF infiltration with the aid of hypodermic needles, while advantageously avoiding injection-associated pain and risk of infection.

The new formulation of deformable liposomes containing 75 micrograms of EGF per gram of substance was applied to a patient with a diabetic foot ulcer, with evidences of compromised blood flow and major amputation risk. The product was administrated topically after complete debriding of the ulcer. The lesion was covered with a dressing and a sterile bandage during the time elapsing between these two treatments. The evolution of the lesion was satisfactory, with evidence of formation of granulation tissue and contraction of ulcer borders from the first week after the start of the treatment. The pharmaceutical product was effective in achieving a complete epithelization of the lesion and preventing foot amputation. There were not side effects associated to the treatment.

Another diabetic patient with ischemic cutaneous lesions in its lower limb with an area exceeding 20 cm² and a depth compromising the periosteum was treated with the traditional liposome-based formulation containing 75 micrograms of EGF per gram of substance. The product was applied topically, three times a week, after complete debriding of the ulcer. The lesion was covered with a dressing and a sterile bandage during the time elapsing between treatments.

After the fourth administration of the formulation, a substantial change in the aspect of the lesion was observed, a productive granulation tissue started to prevail, which in few days was covered by epithelium. At the end of the treatment and total epidermization, the patient was released from the hospital and evolved satisfactorily without any recurrence.

On the other hand, another diabetic patient with advanced ulcers that could not tolerate local injections of EGF and abandoned the infiltration treatment after the third administration, started the administration of the new formulation of deformable liposomes containing 25 micrograms of EGF.

This patient could continue under this treatment with a satisfactory evolution until achieving the complete closure of the lesion. This patient did not report any complication related to its illness or any side effect associated to the treatment.

## Claims

1. Use of an effective amount of epidermal growth factor encapsulated in or associated to deformable or conventional liposomes for manufacturing a topical pharmaceutical formulation for the treatment of grade IV and V chronic ischemic skin lesions in diabetic patients.

2. The use of claim 1, wherein a high bioavailability of epidermal growth factor is achieved in the tissue deep below the lesions.

3. The use of claim 1 and 2, for preventing diabetic foot amputation, wherein the formulation is applied onto the surface and around chronic ischemic skin lesions.

4. A topical pharmaceutical formulation that contains an effective amount of epidermal growth factor encapsulated in or associated to deformable or conventional liposomes, wherein the liposome is comprised of one or more pharmaceutically acceptable lipids, provides a high bioavailability of epidermal growth factor in the tissue deep below chronic ischemic skin lesions for use in preventing diabetic foot amputation.

5. A topical pharmaceutical formulation according to claim 4, wherein the effective amount of epidermal growth factor is in the range from 0.025 to 0.075 mg/gram of substance.

6. A topical pharmaceutical formulation according to claim 4, wherein the effective amount of epidermal growth factor is 0.075 mg/gram of substance.

7. A topical pharmaceutical formulation according to claim 4, wherein the liposome is comprised of one or more pharmaceutically acceptable lipids selected from the group consisting of a neutral lipid, a negatively charged lipid, a positively charged lipid, a lipid conjugated to polyethylene glycol or a lipid conjugated to a carbohydrate.

8. A topical pharmaceutical formulation according to claim 4, wherein the liposome is comprised of one or more pharmaceutically acceptable lipids and one or more non-ionic, zwitterionic, anionic, or cationic surfactant agents.

## Patentansprüche

1. Verwendung einer wirksamen Menge von epidermalem Wachstumsfaktor, eingekapselt in oder assoziiert mit deformierbaren oder konventionellen Liposomen zur Herstellung einer topischen pharmazeutischen Rezeptur für die Behandlung chronischer ischämischer Hautläsion im Stadium IV und V bei Diabetespatienten.

2. Verwendung nach Anspruch 1, wobei eine hohe Bioverfügbarkeit des epidermalen Wachstumsfaktors in dem Gewebe tief unter den Läsionen erzielt wird.

3. Verwendung nach Anspruch 1 und 2 zur Verhinderung einer diabetischen Fußamputation, wobei die Rezeptur auf die Oberfläche und um die chronischen ischämischen Hautläsionen herum angewendet wird.

4. Topische pharmazeutische Rezeptur, die eine wirksame Menge an epidermalem Wachstumsfaktor, eingekapselt in oder assoziiert mit deformierbaren oder konventionellen Liposomen enthält, wobei das Liposom ein oder mehrere pharmazeutisch akzeptable Lipide umfasst, stellt eine hohe Bioverfügbarkeit an epidermalem Wachstumsfaktor in dem Gewebe tief unter chronischen ischämischen Hautläsionenbereit, für die Anwendung zur Verhinderung einer diabetischen Fußamputation.

5. Topische pharmazeutische Rezeptur nach Anspruch 4, wobei die wirksame Menge an epidermalem Wachstumsfaktor im Bereich von 0,025 bis 0,075 mg/g Substanz liegt.

6. Topische pharmazeutische Rezeptur nach Anspruch 4, wobei die wirksame Menge an epidermalem Wachstumsfaktor 0,075 mg/g Substanz ist.

7. Topische pharmazeutische Rezeptur nach Anspruch 4, wobei das Liposom ein oder mehrere pharmazeutisch akzeptable Lipide umfasst, ausgewählt aus der Gruppe, bestehend aus einem neutralen Lipid, einem negativ geladenen Lipid, einem positiv geladenen Lipid, einem an Polyethylenglycol konjugierten Lipid oder einem an ein Kohlenhydrat konjugierten Lipid.

8. Topische pharmazeutische Rezeptur nach Anspruch 4, wobei das Liposom ein oder mehrere pharmazeutisch akzeptable Lipide und ein oder mehrere nicht-ionische, zwitterionische, anionische oder kationische Tensiden umfasst.

## Revendications

1. Utilisation d'une quantité efficace du facteur de croissance épidermique encapsulé dans ou associé à des liposomes déformables ou conventionnels pour fabriquer une formulation pharmaceutique topique destinée au traitement de lésions cutanées ischémiques chroniques de grade IV et V chez des patients diabétiques.

2. Utilisation selon la revendication 1, dans lequel une biodisponibilité élevée du facteur de croissance épidermique est obtenue dans le tissu profondément sous les lésions.

3. Utilisation selon la revendication 1 et 2 pour la prévention de l'amputation du pied diabétique, où la formulation est appliquée sur la surface et autour des lésions cutanées ischémiques chroniques.

4. Formulation pharmaceutique topique qui contient une quantité efficace du facteur de croissance épidermique encapsulé dans ou associé à des liposomes déformables ou conventionnels, où le liposome est constitué d'un ou de plusieurs lipides pharmaceutiquement acceptables, et offre une biodisponibilité élevée du facteur de croissance épidermique dans le tissu profondément sous les lésions cutanées ischémiques chroniques, destinée à être utilisée dans la prévention de l'amputation du pied diabétique.

5. Formulation pharmaceutique topique selon la revendication 4, dans laquelle la quantité efficace du facteur de croissance épidermique est dans la plage comprise entre 0,025 et 0,075 mg/gramme de substance.

6. Formulation pharmaceutique topique selon la revendication 4, dans laquelle la quantité efficace du facteur de croissance épidermique est 0,075 mg/gramme de substance.

7. Formulation pharmaceutique topique selon la revendication 4, dans laquelle le liposome est constitué d'un ou de plusieurs lipides pharmaceutiquement acceptables sélectionnés dans le groupe consistant en un lipide neutre, un lipide négativement chargé, un lipide positivement chargé, un lipide conjugué à du polyéthylène glycol ou un lipide conjugué à un glucide.

8. Formulation pharmaceutique topique selon la revendication 4, dans laquelle le liposome est constitué d'un ou de plusieurs lipides pharmaceutiquement acceptables et d'un ou de plusieurs agents tensioactifs non ioniques, zwitterioniques, anioniques ou cationiques.
